# EUROPÄISCHE PATENTSCHRIFT

(11) **EP 1 261 385 B1**
(45) Veröffentlichungstag und Bekanntmachung des Hinweises auf die Patenterteilung: **08.02.2006**
(21) Anmeldenummer: 01923635.5
(22) Anmeldetag: 05.03.2001
(51) Int. Cl.: A61M 1/10, F04D 1/00, F04D 29/04

(54) **BLUTPUMPE**
BLOOD PUMP
POMPE A SANG

(30) Priorität: 04.03.2000 DE 20004136 U
(43) Veröffentlichungstag der Anmeldung: 04.12.2002
(73) Patentinhaber: Krankenhausbetriebs Gesellschaft Bad Oeynhausen MbH, 33545 Bad Oeynhausen (DE)
(72) Erfinder: SCHULTE EISTRUP, Sebastian, 33442 Herzebrock-Clarholz (DE); BLUDSZUWEIT, Catrin, 18059 Rostock (DE)
(74) Vertreter: Behrmann, Niels
(86) Internationale Anmeldenummer: PCT/EP2001/002480
(87) Internationale Veröffentlichungsnummer: WO 2001/066170

(56) Entgegenhaltungen:
- US-A- 5 713 730
- US-A- 5 924 848

## Beschreibung

Die vorliegende Erfindung betrifft eine Blutpumpe, insbesondere eine ventrikuläre Herzunterstützungspumpe, nach dem Oberbegriff des Anspruches 1.

Zur Therapie von Herzinsuffizienz wurden, neben konservativen, medikamentenbasierten Ansätzen, auch verschiedene chirurgische Therapien entwickelt, von Ersatz oder Rekonstruktion von Herzklappen über elektrische Stimulation des Herzmuskels bis zur Herztransplantation. Aufgrund der limitierten Verfügbarkeit benötigter Spenderherzen und wachsenden Wartelisten kommt jedoch letztere Behandlungsoptiondie gerade in kritischen Fällen größtmöglichen potentiellen Therapie-Erfolg bietet -- einer nur geringen Patientenzahl zugute. So versterben jährlich etwa 20 bis 30 % von zur Herztransplantation angemeldeten Patienten, bevor ein geeignetes Spenderorgan gefunden werden kann.

Die (temporäre) Unterstützung des insuffizienten Kreislaufs durch ventrikuläre Unterstützungssysteme ("VAD"), etwa geeignete Herzunterstützungspumpen, geht zurück bis Anfang der 70iger Jahre und gilt heute als etabliertes und in vielen Transplantationszentren erfolgreich angewandtes Konzept. Dabei existieren neben Systemen zur Unterstützung des linken Ventrikels ("LVAD") insbesondere auch Systeme, die beide erkrankten Ventrikel des Herzes ("BVAD") unterstützen können. Derartige Systeme werden mittlerweile vollständig intrakorporeal implantiert, so dass die Patienten ambulant weiterbehandelt werden können und, insbesondere während der Wartezeit zur Transplantation, nicht mehr an das Krankenhaus stationär gebunden sind.

Konkret bilden ventrikuläre Unterstützungssysteme einen extracardialen Bypass, indem sie Blut aus dem linken Ventrikel in die aufsteigende Aorta pumpen. Im Ergebnis wird der linke Ventrikel entlastet. Wenn entsprechend der rechte Ventrikel ebenfalls insuffizient ist, muss eine diesen umgehende Pumpe entsprechend plaziert werden. VAD werden grundsätzlich so plaziert, dass das native Herz in situ verbleibt, im Unterschied zum sog. Kunstherzen.

Wie neuere wissenschaftliche Veröffentlichungen zeigen, besitzt ein geschädigter, jedoch durch VAD entlasteter Herzmuskel die Fähigkeit zur Regeneration; in einigen Fällen konnte gar auf eine Transplantation verzichtet und das VAD explantiert werden.

Ventrikuläre Unterstützungssystseme bekannter Art werden prinzipiell in Pumpen mit pulsatilem Fluss einerseits sowie mit kontinuierlichem Fluss andererseits unterschieden. Dabei sind pulsatile Herzunterstützungspumpen der Funktionsweise und in dem Rhythmus des nativen Herzens nachempfunden und erzeugen in einem Aktionszyklus sowohl eine Füllungsals auch eine Austreibungsphase für Blut mittels einer Blutkammer, die typischerweise aus einem elastischen Kunststoffsack besteht, wobei Klappen für einen gerichteten Blutstrom sorgen. Derartige Systeme haben sich klinisch bewährt und ermöglichen die Unterstützung von Patienten in Zeiträumen von bis zu etwa zwei Jahren. Gleichwohl weisen pulsatile Herzunterstützungssysteme auch einige Nachteile auf. So sind derartige VAD üblicherweise voluminös und nur schwer im Patientenkörper problemlos zu implantieren. Auch ist die Effizienz pulsatiler VAD durch den komplizierten Antriebsmechanismus sehr niedrig, und das Antriebs- und Steuerungssystem ist komplex und damit gerade bei Langzeitimplantationen störungsanfällig. Vor allem jedoch führt die lange Kontaktzeit des Blutes während der Füllungsphase vermehrt zu Thromben, die dann die Gefahr zentraler Embolien mit neurologischen Ausfällen od.dgl. bewirken; unter stützt wird diese Gefahr der Thrombusbildung durch an den Klappen entstehende Turbulenzen mit vermehrtem Scherstress im Blutfluss.

Non-Pulsatile VAD generieren im Gegensatz zur vorbeschriebenen Technologie einen kontinuierlichen Blutfluss und benötigen eine relativ kleine Blutkammer ohne elastische Auskleidung und Herzklappen. Entsprechend läßt sich die Größe dieser Unterstützungspumpen erheblich reduzieren, was sie einem größeren Patientenkreis zugänglich macht. Auch besteht durch das Fehlen einer elastischen Membrane innerhalb der Blutkammer bzw. von Herzklappen eine deutlich reduzierte Thrombosegefahr. Da zudem das Antriebssystem nonpulsatiler VAD einfach und effizient ist, kommen herkömmliche, bürstenfreie DC-Motoren zum Einsatz, mit einem Energieverbrauch der unterhalb 8 Watt und damit niedrig liegt.

Die non-pulsatile Kreislaufunterstützung bringt jedoch andere Schwierigkeiten mit sich. Durch das Fehlen von Herzklappen (die einen gerichteten Blutfluss ermöglichen) besteht im Falle eines Pumpenversagens die Gefahr rezirkulierenden Blutes, die durch zusätzliche Maßnahmen behoben werden muss. Auch ist die Förderleistung eines non-pulsatilen VAD schlecht zu ermitteln, da keine Blutkammer mit definiertem Volumen vorhanden ist, so dass genauere Aussagen über die Förderleistung lediglich durch exakte Flussmessungen (etwa mit implantiertem Flussmeter) gemacht werden können.

Non-pulsatile Blutpumpen lassen sich in Zentrifugal- und Axialpumpen unterscheiden, wobei letztere technologisch zur Zeit keine größere Rolle im praktischen klinischen Einsatz spielen, so dass im folgenden ausschließlich die Zentrifugalpumpe als relevanter Stand der Technik (sowie als Gattung für die vorliegende Erfindung) diskutiert wird.

Handelsübliche Zentrifugalpumpen zur Herzunterstützung beschleunigen das Blut rechtwinkelig zur Richtung des einströmenden Blutflusses und weisen üblicherweise eine konusförmige Blutkammer auf, in welcher ein (üblicherweise magnetisch gekoppelter) Rotor drehbar aufgehängt ist. Traditionell wird das Blut der Spitze der Blutkammer durch eine Einflusskanüle zugeführt, über den Rotor gleichmäßig verteilt und zentrifugal beschleunigt, und Blut verlässt im Bereich des größten Druckes und größter Geschwindigkeit die Blutkammer durch eine Ausflusskanüle, deren Achse üblicherweise rechtwinkelig zur Rotorachse verläuft. Die Beschleunigung des Blutes erfolgt üblicherweise durch am Rotor vorgesehene Rotorblätter. Allerdings entsteht bei derartigen, bekannten Zentrifugalpumpen mit konusförmiger Blutkammer ein relativ komplexes Flussmuster des geförderten Blutes mit einem inversen Druckverhältnis auf der Rückseite des Rotors, mit der Gefahr, dass gerade im Bereich der Rotorwelle (der Begriff "Welle" soll im Rahmen der vorliegenden Anmeldung auch als Synonym mit "Achse" verstanden und verwendet werden), an seiner Aufhängung bzw. am Rotordach die Gefahr von Thrombenbildung besteht. So würde ein an der Pumpenwelle wachsender Thrombus in Umfangsrichtung wachsen können und zu einer kontinuierlichen Reibungszunahme führen, mit deutlich erhöhtem Energieverbrauch und dem Risiko eines Pumpenarrests; darüber hinaus besteht die Gefahr austretender Thrombus- bzw. Emboliebildung für das System des Patienten.

Die US 5,924,848 offenbart eine gattungsgemäße Blutpumpe mit den oberbegrifflichen Merkmalen des Anspruches 1, wobei hier die Blutkammer, im Gegensatz zum vorbeschriebenen Stand der Technik, als Doppelkonus mit zwei einander gegenüberliegenden Einlässen an jeweiligen Spitzen des Doppelkonus realisiert ist. Diese Technologie sieht einen Rotor vor, der in der Blutkammer im Pumpengehäuse lagerfrei rotieren kann und durch hydrodynamische Kräfte in Position gehalten wird. Wie der Erfinder dazu erläutert, werden durch eine solche Lösung, neben gesteigerter Effizienz, vor allem auch Probleme der Lagerung sowie wiederum daraus resultierende Thrombenbildung usw. verhindert.

Allerdings erscheint auch diese bekannte Lösung nicht optimal und, insbesondere im praktischen klinischen Betrieb unter wechselnden Einsatzbedingungen, potentiell problematisch. So ist nämlich davon auszugehen, dass etwa Lageveränderungen eines Patienten (mit implantierter Pumpe) zu Druckänderungen und damit Flussänderungen an den Einlässen der Pumpe führen, und auch zu nicht unerheblichen mechanischen Belastungen auf den Rotor; aufgrund der (effizienzbedingt) geringen Abstände zwischen den Spitzen der Rotorblätter und dem Pumpengehäuse (etwa 1 bis 3 mm) kann es somit leicht zu Kollisionen des Rotors mit dem inneren Pumpengehäuse kommen, mit der Gefahr von Beschädigungen, erhöhtem Verschleiss sowie Ausfall.

Aufgabe der vorliegenden Erfindung ist es daher, eine bekannte Blutpumpe, insbesondere zur ventikulären Herzunterstützung, dahingehend zu verbessern, dass die praktischen Betriebseigenschaften gegenüber bekannten Pumpenlösungen verbessert werden, insbesondere die Eignung für verschiedene Betriebs- und Patientenbedingungen erhöht und das Risiko schädlicher Thrombusbildung vermindert werden kann. Gleichzeitig soll eine neue, zu schaffende Pumpe sich durch geringen Verschleiss, hohe Zuverlässigkeit und niedrigen Energieverbrauch auszeichnen.

Die Aufgabe wird durch die Blutpumpe mit den Merkmalen des Anspruches 1 gelöst; vorteilhafte Weiterbildungen der Erfindung sind in den Unteransprüchen beschrieben.

So ist erfindungsgemäß vorgesehen, dass die Rotorachse (Rotorwelle) endseitig in mechanisch wirksamen Lagern drehbar gelagert ist, wobei die Lagerung am Pumpengehäuse jeweils im Bereich der gegenüberliegenden Einlässe erfolgt. Mit anderen Worten, gemäß der Erfindung erfolgt also eine feste Lagerung der Rotorwelle jeweils im Bereich einfließenden Blutes, so dass auf ein jeweiliges, gelagertes Ende der Rotorwelle ein entgegengesetzter Blutstrom einfließenden Blutes trifft. Dies führt erfindungsgemäß und vorteilhaft dazu, dass eine axiale Belastung der Lager im Idealzustand praktisch nicht erfolgt, denn eine durch einfließendes Blut in einen ersten Einlass ausgeübte axiale Kraft auf den Rotor (bzw. die Rotorwelle) wird durch die entgegengesetzt wirkende, durch einfließendes Blut in den zweiten Einlass erzeugte Kraft wiederum aufgehoben. Das Ergebnis ist (verglichen mit dem Stand der Technik, wo einseitig an einer Konusspitze eintretendes Blut durch eine Axialkraft herkömmliche Lager erheblich belastet) eine äußerst geringe Verschleissanfälligkeit, so dass von hoher Langlebigkeit und geringer Störanfälligkeit des erfindunsgemäßen Lagerungsprinzips ausgegangen werden kann. Gleichzeitig vermeidet die vorliegende Erfindung jedoch die Unwägbarkeiten und das Störungsrisiko durch lagerfreie Aufhängung des Rotors wie bei der gattungsbildenden US 5, 924 ,848, ebenso wie -- im Fall einer rein mechanischen Lagerung ohne Magnetunterstützung -- zusätzlichen elektrischen Energieverbrauch.

Ungeachtet dessen ist es im Rahmen einer möglichen Weiterbildung vorgesehen, die erfindungsgemäß primär mechanisch wirksamen Lager durch Magnete (Permanent- oder Elektromagnete) zu unterstützen, wobei es beispielsweise möglich ist, durch geeignete Dimensionierung der mechanischen Lagerung eine zwar primär magnetisch wirksamen Führung der Rotorachse (entsprechend reibungs- bzw. verschleißarm) zu realisieren, gleichzeitig jedoch die nach wie vor vorhandenen mechanischen Lageraggregate dann wirksam werden zu lassen, wenn, etwa durch Erschütterungen oder andere, potentiell für reine Magnetlagerungen problematische Zustände, der Rotor aus einer stationären Mittellage bewegt wird, so dass in diesem Fall der mechanisch wirksamen Lagerung eine Sicherungswirkung zukommt.

Weiterbildungsgemäß ist vorgesehen, den Blutfluss durch die Pumpe äußerst effizient, gleichwohl verwirbelungsarm und bei Verminderung möglicher Scherkräfte auf den Blutstrom (wodurch die Gefahr von Thrombenbildung entsteht) schonend zu gestalten.

Zum einen wird dies durch geeignete Ausgestaltung der Einfluss- und Ausflussgeometrie dergestalt ermöglicht, dass die für eine Flussgeschwindigkeit an den Einlässen maßgebliche Querschnittsfläche größer ausgebildet ist als eine entsprechende Querschnittsfläche des Auslasses (während dagegen natürlich die Querschnittsfläche des Ausflussstutzens gleich der Fläche des der Pumpe vorgeschalteten intraventikulären singulären Zuflusses sein muss). Durch die weiterbildungsgemäße Vergrößerung des Einflussquerschnittes wird nämlich der Blutfluss verlangsamt, mit der Folge, dass weniger Bluttrauma am Einlass sowie an der Rotoroberfläche entsteht. Auch muss -- durch die gegenüberliegenden Einlässe konstruktionsbedingt -- das Blut durch relativ lange Einflusskanülen zugeführt werden, die selbst wiederum einen Druckabfall bedingen.

Darüber hinaus ist weiterbildungsgemäß vorgesehen, in den Einlässen Mittel zur Flussleitung vorzusehen, die vorzugsweise zur Vorrotation, zur Wirbelverhinderung oder aber als Flussbegradiger wirken. Auch hierdurch wird die Gefahr schädlicher Thrombenbildung weiter herabgesetzt, wobei z. B. erfindungsgemäß geeignet ausgestaltete Einrichtungen zur Flussführung etwa Flussbegradiger, gleichzeitig zur zuverlässigen und effizienten endseitigen Aufhängung bzw. Lagerung des Rotors (der Rotorwelle) im Rahmen der vorliegenden Erfindung genutzt werden können. Eine weitere, bevorzugte Weiterbildung sieht zudem vor, eine derartige Lagerung durch einfließendes Blut zu spülen, indem ein geeigneter Vorsprung, besonders in der Art einer Spüllippe, einen Teil einfließendes Blutes zu Spülzwecken in das Lager einleitet.

Als weitere erfindungsgemäße Maßnahme zur schonenden, wirbelarmen Beschleunigung des Blutes ist vorgesehen, neben den zur unmittelbaren Zentrifugalbeschleunigung eingesetzten Rotorblättern auch die Relativgeometrie zwischen Pumpeninnenraum und Rotorkörper zu benutzen. Genauer gesagt ist der Rotorkörper mit seinen Außenflächen so ausgebildet, dass ein wirksamer (Fluss-) Querschnitt für fließendes Blut zwischen Einlässen und Auslass in Richtung auf den Auslass abnimmt; beispielsweise kann dies dadurch realisiert werden, dass ein (ebenfalls) doppel-konusförmiger Rotorkörper eine größere Konussteigerung besitzt als die umgebenden Wände der Blutkammer. Dies führt dann dazu, dass einfließendes Blut durch Scherkräfte am Rotorkörper zusätzlich bewegt wird und die gesamte Energieübertragung der Rotorenergie auf das Blut mit hoher Effizienz bei niedrigem Druck erfolgt; entsprechend können die Rotorblätter -- vor allem in axialer Richtung -- verkleinert werden, ohne die Gesamteffizienz der Pumpe zu mindern. Eine Verkleinerung der Rotorblätter wiederum (die weiter bevorzugt im Querschnitt endseitig gerundet sind) vermindert wiederum die Gefahr von Blutverletzung, insbesondere als der etwa aus dem Stand der Technik gemäß US 5,924,848 ersichtliche "Mühleneffekt" (der weitgehend bündig mit der Blutkammer abschließende Rotor verhält sich für einfließendes Blut ähnlich einer Mühle und kann Blutbestandteile zerstören; gleichzeitig würde Blut im Einflussstutzen zentrifugal beschleunigt und kann zu Füllungsbehinderungen mit reduzierter Effizienz führen) vermieden wird. Vielmehr beginnt eine beschleunigungswirksame Rotorfläche erst in einem gewissen, vorbestimmten Abstand von den jeweiligen Einlässen, so dass Blut laminar in die Blutkammer eintreten und parallel der Welle fließen kann. Das Ergebnis ist deutlich geringeres Bluttrauma, jedoch, durch die vorbeschriebenen geometrischkonstruktiven Maßnahmen, gleichwohl hohe Effizienz des Pumpensystems. Zusätzlich verhindern die weiterbildungsgemäß vorgesehenen Flussbegradiger eine (rotorbedingt) schädliche Zentrifugalströmung des Blutes im Bereich der Einlässe.

Gemäß einer weiteren, vorteilhaften Weiterbildung ist vorgesehen, die für den Antrieb notwendigen Magneten des Rotors nicht an den Rotorblättern selbst, z.B. an deren Spitzen, vorzusehen, sondern im Rotorkörper selbst. Durch eine solche Maßnahme wird die Balancierung des Rotors, insbesondere für hohe Drehzahlen, deutlich vereinfacht und die radiale Lagerbelastung vermindert; etwaige Effizienzeinbußen werden wiederum durch die vorbeschriebenen konstruktiven Maßnahmen in der verwirbelungsarm ausgerichteten Rotorgeometrie ausgeglichen. Vorteilhaft ist es hier zudem, dass ein Stabmagnet vorgesehen ist, welcher bevorzugt im Inneren des Rotorkörpers, benachbart zu dessen Außenfläche und senkrecht zur Rotationsachse vorgesehen sein kann und sich z. B. von einer Außenfläche zur gegenüberliegenden Außenfläche des Rotorkörpers erstreckt.

Gemäß einer anderen, vorteilhaften Weiterbildung der Erfindung ist vorgesehen, die mechanisch wirksamen Lager durch jeweils geeignet ausgebildete Gehäuse zu verkapseln, wobei auch hier bevorzugt eine Gehäuseform gewählt wird, die strömungsgünstig an jeweils in die Einlässe einfließendes Blut angepasst ist, dieses sogar für eine Kühlung der jeweiligen Lager nutzt. Die besonders bevorzugten Gleit- oder Wälzlager sind jeweils eingebettet in das gut wärmeleitende Lagergehäuse, welches gleichzeitig der Fixierung der Lager, ihrer Abgrenzung vom Strömungsgebiet, einer optimalen, atraumatischen Umströmung der Lager und der Wärmeableitung vom Lager dient. Das Lagergehäuse wird mittels Strömungsleiteinrichtungen am Pumpengehäuse befestigt. Die Gestaltung des Lagergehäuses erfolgt derart, dass eine geringe Beeinflussung der axialen Zuströmung erfolgt. Die Strömung folgt der zu den Enden spitz zulaufenden Form des Lagergehäuses, ohne blutschädigende Unregelmäßigkeiten oder Stagnationsgebiete zu bilden. Eine solche Realisierung mit Gleit- oder Wälzlagern innerhalb eines gut wärmeleitenden Lagergehäuses kann dann, wie vorbeschrieben, mittels geeignet ausgebildeter Strömungsleiteinrichtungen am Pumpengehäuse befestigt sein, wobei, weiter bevorzugt, die Rotorachse gegen ein stehendes Lagergehäuse durch eine aus geeignetem, bevorzugt verschleißfreiem Material realisierte Dichtung abgedichtet wird. Die Dichtung kann verschiedene Formen annehmen und an verschiedenen Positionen angebracht sein. Eine spitz auslaufende Dichtung kann z.B. direkt an der zylindrischen Welle anliegen. Eine mehr flächenhafte Pressung der Dichtung kann z.B. durch eine spezielle Wellenform bzw. einen fest mit der Welle verbundenen Aufsatz erfolgen. Auch hier erfolgt bevorzugt die Dimensionierung von Gehäuse bzw. Dichtung ohne negative Auswirkungen auf die Blutströmung, wobei insbesondere der beabsichtigte Kühleffekt aufgrund der hohen Blutgeschwindigkeiten sehr effektiv ist.

Eine weitere, bevorzugte Ausführungsform der Erfindung sieht vor, eine Mehrzahl von Pumpenausflüssen bzw. -auslässen vorzusehen, wobei, insbesondere, wenn diese mit ihren jeweiligen Öffnungen zur Blutkammer radial um deren Umfang herum angeordnet sind, eine Belastung des Rotors etwa durch Strömungseffekte bzw. Strömungswiderstände (im Gegensatz zu einer einzigen Öffnung) verringert werden kann; besonders geeignet sind diese Auslässe mittels spiralförmiger Gehäuseerweiterungen eines Blutpumpengehäuses realisiert.

Besonders vorteilhaft ist es zudem, die vorteilhaften Betriebs- und Herstellungseigenschaften der erfindungsgemäßen Blutpumpe durch Konfigurationen des Pumpenantriebs zu unterstützen, wobei, gemäß einer bevorzugten Ausführungsform (best mode) der Rotor einen geschlossenen Rotorkörper aufweist, von dessen Außenflächen sich eine Mehrzahl von bevorzugt radial um einen Umfang des Rotorgehäuses angeordneten Rotorblättern erstreckt, und wobei elektrische und/oder magnetische Antriebselemente des Rotors im Inneren des Rotorkörpers, bevorzugt benachbart den Außenflächen des Rotorkörpers, fest aufgenommen und zum Zusammenwirken mit im die Blutkammer umschließenden Pumpengehäuse vorgesehenen elektrischen und/oder magnetischen Antriebselementen der Blutpumpe ausgebildet sind. Insbesondere bietet es sich vorteilhaft an, Spulen als elektrische Antriebselemente konstruktiv so auszuführen, dass darin (durch elektrischen Widerstand) erzeugte Wärme möglichst günstig abgeführt wird, bevorzugt durch den Blutstrom. Dies erfolgt einerseits durch einen engen Kontakt zur blutseitigen Gehäusewand und einer thermischen Isolierung durch Luft oder einen Füllstoff an der Rückseite der Spule. Zwischen Spule und thermischer Isolierung kann sich eine Ummantelung aus ferromagnetischem Material zur Konzentrierung des Magnetfeldes befinden. Weiter bevorzugt ist es, Spulen mit einer Mehrzahl von Wicklungen vorzusehen und diese entweder zum Zweck der Redundanz separat ansteuerbar auszubilden, oder aber die separaten Einzelwicklungen für Zwecke der Leistungssteuerung der Pumpe durch eine geeignet zugeordnete Steuerelektronik ansteuerbar zu gestalten.

Aufbauend auf die Ansteuerung bzw. Leistungssteuerung der Pumpe bestehen zudem zahlreiche, vorteilhafte Weiterbildungsmöglichkeiten, etwa dahingehend, dass die erfindungsgemäße Blutpumpe gemäß einer weiteren vorteilhaften Weiterbildung als geregeltes System verstanden wird. Die weiterbildungsgemäß vorgesehenen Mittel zur Blutstrom-, Druck- und/oder Flussmessung des Blutes können dabei bevorzugt mit Mitteln für eine Drehzahl- und/oder Leistungserfassung der Pumpe zusammenwirken, so dass, durch geeignetes Zusammenführen entlang bzw. auf der Grundlage einer Pumpenkennlinie, eine eigenständige Belastungsadaption an den jeweiligen Zustand des Patienten, wie durch die Parameter des fließenden Blutes gemessen, möglich ist.

Letztendlich steht hinter diesem Ansatz ein umfassendes, transkutanes Energie- und Informationstransfersytem, welches zudem, etwa mit Hilfe von bevorzugt drahtlosen Kommunikationsmitteln, auch eine Kommunikation mit externen Überwachungs- und Steueraggregaten ermöglicht. Damit werden dem Patienten größtmögliche Freiräume ermöglicht, ohne die notwendige medizinische Kontrolle zu verlieren.

Im Rahmen der Möglichkeiten zur Weiterbildung der Erfindung durch geeignete Konfiguraton einer zugeordneten Steuerelektronik ist es zudem denkbar, weitere relevante Steuerparameter, wie etwa EKG-Signale, die weiterbildungsgemäß besonders bevorzugt durch einen etwaigen, vorhandenen Herzschrittmacher ohnehin erfasst werden und für eine elektronische Bearbeitung zur Verfügung stehen, zu berücksichtigen und somit für eine integrierte Pumpenansteuerung zu sorgen, die in weitgehender Weise von aktuellen Parametern bzw. Funktionswerten des zu unterstützenden Herzens abhängig ist; für die im Rahmen der vorliegenden Erfindung mögliche Kombination einer Pumpenansteuerung mit einer Steuereinheit für einen Herzschrittmacher wird, über die konkrete Anwendung im Rahmen der vorliegenden Erfindung hinaus, auch unabhängig Schutz beansprucht.

Im Ergebnis ermöglicht es die vorliegende Erfindung, gängige Pumpen zur non-pulsatilen Herzunterstützung sowohl im Hinblick auf Betriebssicherheit, als auch potentielle Gefährdung des Patienten durch Thrombenbildung deutlich zu verbessern, ohne dass konstruktiv übergroßer Aufwand notwendig ist. Vielmehr zeichnet sich die vorliegende Erfindung durch zusätzlich einfache Herstellbarkeit und unproblematisches mechanisches Toleranzverhalten aus, so dass davon auszugehen ist, dass mit der vorliegenden Erfindung zusätzliche Patientenkreise und Anwendungsgebiete erschlossen werden können.

Weitere Vorteile, Merkmale und Einzelheiten der vorliegenden Erfindung ergeben sich aus der nachfolgenden Beschreibung bevorzugter Ausführungsbeispiele sowie anhand der Zeichnungen; diese zeigen in:
- Fig. 1:: eine schematische seitliche Schnittansicht in axialer Richtung der Rotorachse einer ersten bevorzugten Ausführungsform der vorliegenden Erfindung (best mode);
- Fig. 2:: eine schematische Ansicht eines Längsschnittes durch die Blutkammer der Ausführungsform gemäß Fig. 1;
- Fig. 3:: eine Schnittansicht entlang der Schnittlinie III-III in Fig. 3;

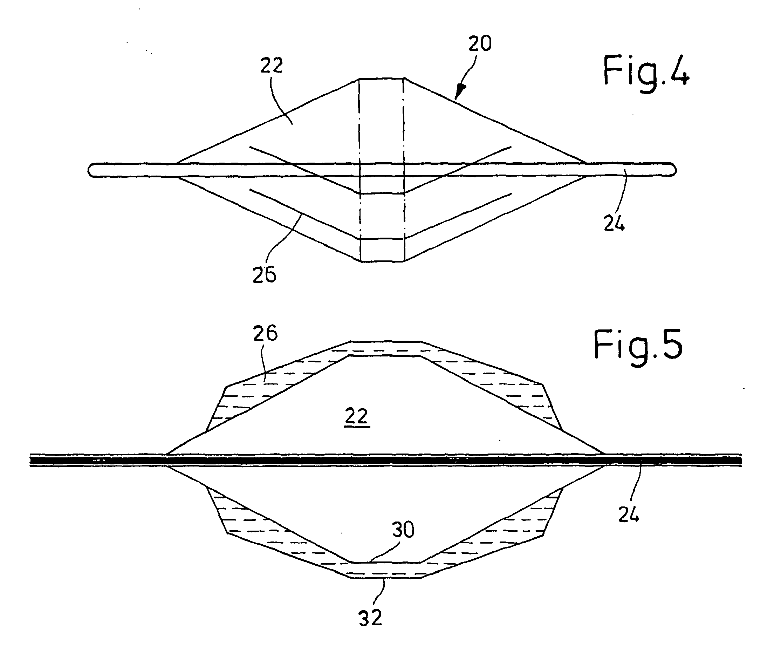

- Fig. 4:: eine schematische Seitenansicht des Rotors der Ausführungsform gemäß Fig. 1;
- Fig. 5:: eine Schnittansicht durch den Rotor;
- Fig. 6:: eine Seitenansicht eines einlassseitigen Flussbegradigers zur Verwendung mit der Ausführungsform gemäß Fig. 1;
- Fig. 7:: eine Ansicht des (eingesetzten) Flussbegradigers gemäß Fig. 6 aus einlassseitiger Richtung;
- Fig. 8:: eine schematische Seitenansicht einer Wellenlagerung im Einlassbereich mit Spüllippe und schematisch angedeuteter Umleitung des Blutflusses zu Spülzwecken;
- Fig. 9:: eine Schnittansicht innerhalb der Schnittlinie IX-IX in Fig. 8;
- Fig. 10:: eine schematische Ansicht von links- und rechtsseitigen Einlasskanülen zur Verbindung der Pumpe gemäß Fig. 1 mit der gemeinsamen Einlasskanüle des Ventrikels;
- Fig. 11:: eine schematische seitliche Schnittansicht einer alternativen Ausführungsform mit gekapstelten, gedichteten Lagergehäusen zur Lagerung der Rotorachse und
- Fig. 12:: eine schematische Schnittansicht durch den Rotorkörper bzw. das Pumpengehäuse zum Verdeutlichen einer alternativen Ausführungsform des Auslasses mit einer Mehrzahl spiralförmig angeordneter, mit ihren jeweiligen Öffnungen zur Blutkammer umfangsseitig um die Blutkammer herum angeordneten Kanälen.

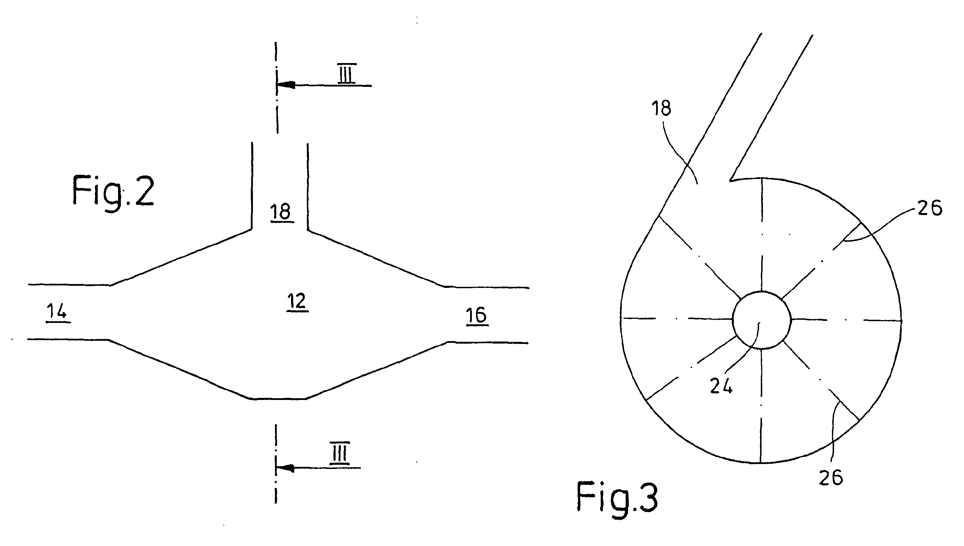

Eine schematisch mit zylindrischem Pumpengehäuse 10 gezeigte Blutpumpe gemäß einer ersten Ausführungsform der vorliegenden Erfindung weist im Pumpengehäuse 10 eine in der gezeigten axialen Schnittansicht i.w. oktogonale Blutkammer 12 auf, die in der Art eines Doppelkonus beidends an spitzen Enden einen linken 14 bzw. rechten Einlass 16 ausbildet und, wie insbesondere in der Schnittansicht der Fig. 3 zu erkennen ist, im mittleren Bereich (nämlich dem größten Durchmesser des Doppelkonus) in einen sich in Umfangsrichtung i.w. tangential erstreckenden Auslass mündet.

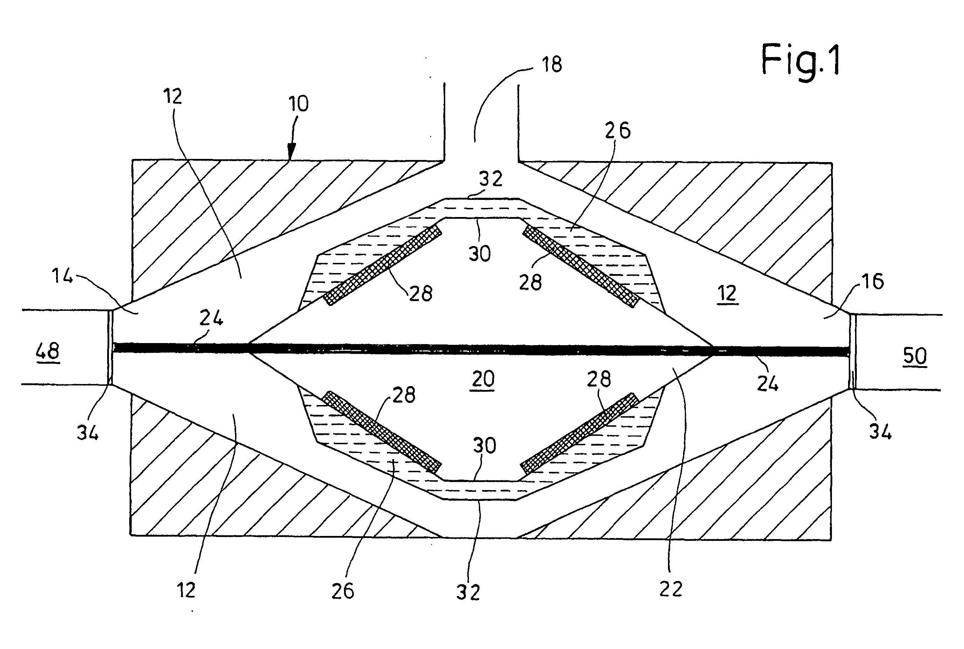

Wie schematisch in der Ansicht der Fig. 1 zu erkennen ist, wird ein Rotor 20, bestehend aus einem ebenfalls doppelkonusförmigen Rotorkörper sowie einer Rotorwelle (Rotorachse) 24, die sich durch die gegenüberliegenden spitzen Endabschnitte der Rotorkörpers 22 erstreckt, in der Blutkammer 12 im Bereich des linken Einlasses 14 sowie des rechten Einlasses 16 gelagert, und zwar so, dass durch die Einlässe 14, 16 einströmendes Blut die Rotorwelle 24 endseitig i.w. gleichförmig umströmen kann, auf den Rotorkörper 22 trifft und sowohl durch Wirkung von sechs radial um den Umfang des Rotorkörpers 22 verteilt angeodneten Rotorblättern 26 zentrifugal nach außen getrieben wird, als auch durch einen sich in Richtung auf den Auslass 18 kontinuierlich verringernden lichten bzw. freien Strömungsquerschnitt zwischen Pumpengehäuse 10 und Rotorkörper 22 in Auslassrichtung beschleunigt wird.

Der Antrieb des Rotors 22 erfolgt dabei durch Rotormagnete 28, die innerhalb des Rotorkörpers 22 unter dessen Oberfläche angeordnet sind und mit Antriebselementen eines Pumpenmotors zusammenwirken, die -- in den Figuren nicht gezeigt -- im Pumpengehäuse den Rotormagneten 28 gegenüberliegend vorgesehen sind.

Durch die vorbeschriebenen und in Fig. 1 gezeigten geometrischen Verhältnisse ermöglicht die mechanische Ausgestaltung der Blutpumpe der gezeigten Ausführungsform eine besonders schonende, atraumatische, gleichwohl effiziente Förderung von durch die Einlässe 14, 16 eintretenden Blutes: Wie insbesondere in der Fig. 1 gut erkennbar ist, beginnt der doppel-konusförmige Rotorkörper 22 mit seinen jeweiligen zulaufenden Endabschnitten erst einen vorbestimmten Abstand von einem jeweiligen zugeordneten Einlassende; bei einem typischen Außendurchmesser des Pumpengehäuses von 35 mm und einer beispielhaften Länge von ca. 70 mm beträgt dieser Abstand auf jeder Seite ca. 10 bis 12 mm. Dieser Abstand ermöglicht es dann, dass durch die jeweiligen Einlässe eintretendes Blut zunächst verwirbelungsarm und i.w. laminar in Richtung der Rotorwelle einfließen kann, und insbesondere Effekte einer einlassseitigen Zentrifugalbeschleunigung bzw. eines Zermahlens von Blutbestandteilen durch Rotorwellenbewegung am jeweiligen Einlass vermieden werden. Eine weitere Maßnahme, die sich positiv auf die atraumatische Wirkung der gezeigten Anordnung auswirkt, besteht in der Gestaltung der linken bzw. rechten Einlassgeometrie relativ zum Auslass: Wie in der Fig. 1 erkennbar ist, ist der kumulierte Einlassquerschnitt beider Einlässe 14, 16 größer als der wirksame Flussquerschnitt des Auslasses 18 (bzw. des wirksamen ventrikulären Zuflussquerschnittes). Dies bedeutet, dass durch eine graduelle Erweiterung eines Flussquerschnittes einer angeschlossenen Einflusskanüle zum Einlass hin der Blutfluss graduell verlangsamt wird und somit sich weniger traumatisch auf das Blut auswirkt.

Wie zudem in der Fig. 1 erkennbar ist, ist sowohl eine Abflachung 30 im mittleren Bereich des Rotorkörpers 22 als auch eine Abflachung 32 im peripheren Endbereich der Rotorblätter 26 gebildet, wobei in axialer Richtung diese Abflachungen 30, 32 jeweils der axialen Erstreckung des Auslassquerschnittes entsprechen; auch diese Maßnahme dient der atraumatischen Flussoptimierung in der beschriebenen Ausführungsform.

In erfindungsgemäß vorteilhafter Weise ist die Rotorwelle 24 beidends in (in Fig. 1 lediglich schematisch gezeigten) Lagern 34 drehbar gelagert, wobei, bedingt durch den symmetrischen Blutzufluss durch beide Einlässe 14, 16, eine beidseitig auf den Rotor 20 ausgeübte Axialkraft sich bei identischen Drücken einfließenden Blutes im Idealfall aufhebt, im Normalfall lediglich geringer Druckdifferenzen jedoch zu einer äußerst geringen axialen Belastung des Lagerpaares 34 führt. Das Ergebnis ist eine äußerste Langlebigkeit der nachfolgend noch im Detail zu beschreibenden Lageranordnung. Lediglich der Vollständigkeit halber sei erläutert, dass auch die in Fig. 1 gut erkennbar Anordnung der Magneten 28 im Rotorkörper 22 eine einfache und exakte Ausbalancierung des Rotors in Radialrichtung ermöglicht, so dass auch in dieser Richtung die auf die Lager 34 wirkenden Belastungen minimiert sind.

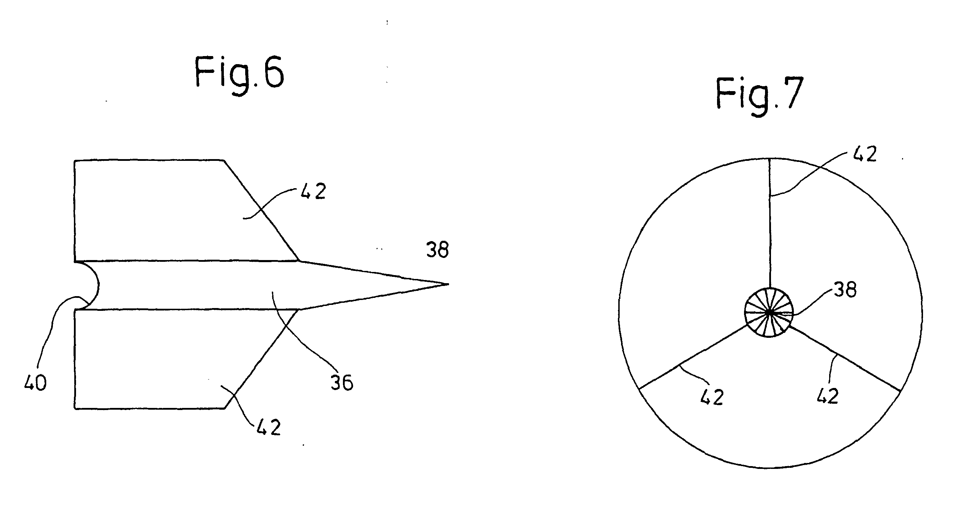

Die Fig. 6 und 7 zeigen gemäß einer bevorzugten Weiterbildung einlassseitig vorsehbare Flussbegradiger 36 als Strömungsleiteinrichtung, die in der Seitenansicht der Fig. 6 eine in Richtung auf den einfließenden Blutfluss zulaufende Spitze 38 aufweisen, am entgegengesetzten Ende eine Lagerschale 40 zur Aufnahme der Rotorwelle 24 ausbilden und drei um den Umfang des Begradigerelements 36 verteilte, sich radial erstreckende Finnen 42 tragen, die, wie insbesondere aus der Sicht der Zuflussrichtung gemäß Fig. 7 zu erkennen ist, zusammen mit der Spitze 36 einen lediglich minimalen Strömungswiderstand für den einfließenden Blutstrom bilden, dagegen in Umfangsrichtung das Entstehen von Verwirbelungen od.dgl. (insbesondere auch durch Wirbeleffekte an einem jeweiligen Einlass) wirksam verhindern können.

Die Frontfläche der Finnen weisen einen sich verjüngenden Verlauf auf, während im dargestellten Ausführungsbeispiel die Rückflächen bündig mit dem zentralen Trägerelement des Flussbegradigers 36 abschließen. Die sich verjüngenden Frontflächen eignen sich besonders zur Vermeidung von Thrombusbildungen, als der Blutfluss geordnet und verwirbelungsarm um das zentrale Trägerelement des Flussbegradigers 36 und die Finnen herumgeleitet wird, und durch die gezeigte Geometrie existieren lediglich minimale Bereiche mit Umkehrflüssen und Blutverwirbelungen hinter dem Flussbegradiger.

Konkret wird, wie in der Fig. 7 angedeutet, ein Flussbegradiger 36 in jeden der Einlässe 14, 16 eingeführt, kontaktiert dort das Pumpengehäuse 12 und bietet gleichzeitig mittels der Lagerschale 40 ein symmetriebedingt wenig belastetes und damit überaus dauerhaftes Lager für die Pumpenwelle an.

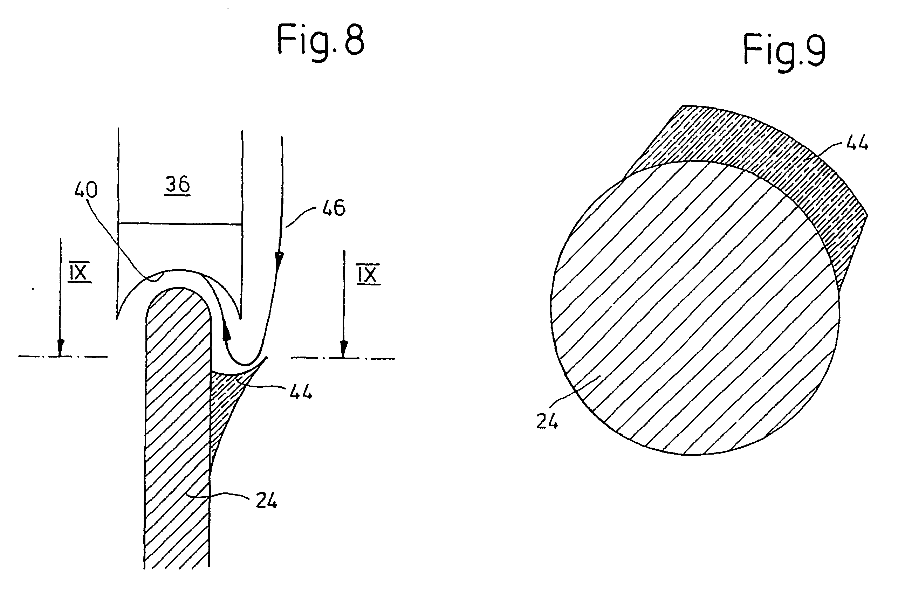

Gemäß einer weiteren vorteilhaften Weiterbildung der Erfindung ist es zudem möglich, dass zwischen Lagerschale 40 und gerundetem Ende der Rotorwelle 24 gebildete Lager durch einfließendes Blut zu spülen: Wie die Fig. 8 und 9 zeigen, ist nämlich endseitig an der Rotorwelle 24 ein Vorsprung in der Art einer Spüllippe 44 gebildet, der, wie durch die Strömungslinie 46 verdeutlicht ist, einen Teil des durch einen Einlass einströmenden Blutes in Richtung auf die Lagerschale 40 umlenkt und so für eine zuverlässige Spülung des Lagers sorgt.

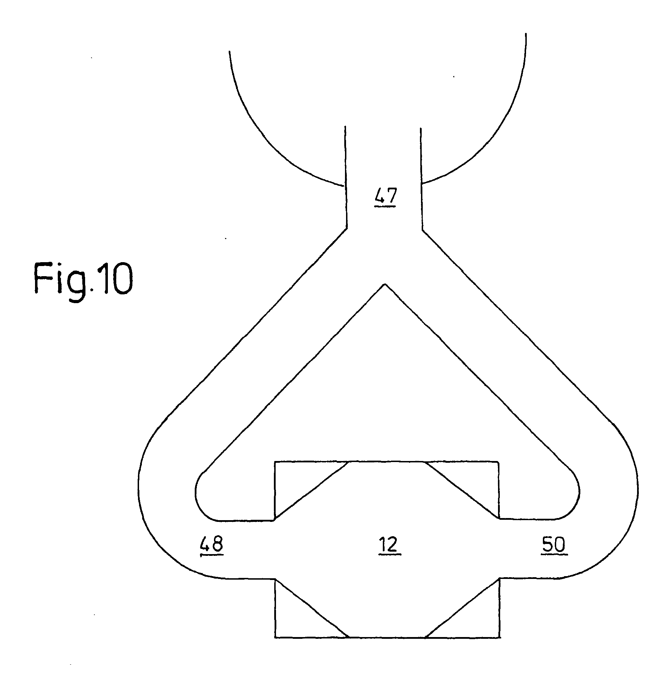

Die Fig. 10 zeigt die einlassseitige Verbindung des Pumpengehäuses 10 mit dem Ventrikel mittels einer links- und einer rechtsseitigen Einlasskanüle 48, 50, die, zur Verminderung eines Druckabfalls, im Körper des Patienten in einem relativ großen Radius geführt, und bevorzugt als starres System (alternativ: flexibel oder kombiniert aus beiden) ausgebildet sind.

Wie in Fig. 10 gezeigt, besteht konkret die Einflusskanüle aus einer großkalibrigen, singulären Kanüle 47, die in den linken Ventrikel eingebracht wird und einen (nicht gezeigten) Flussteiler aufweist. Im Einsatz unmittelbar unterhalb des Zwerchfelles bzw. Herzens implantiert, teilt sich diese Kanüle 47 so in die zwei gleichkalibrigen, gleichlangen Einlasskanülen 48, 50 auf, die das Blut in möglichst großem Bogen der Pumpe und Blutkammer zuführen. Dabei sollte die Aufteilung der gemeinsamen Kanüle 47 so spitz wie möglich sein, um eine homogene Aufteilung des Blutes zu erreichen, damit wiederum die Pumpe mit nahezu gleichem Blutvolumina und Drücken an dem linken Einlass 14 und rechten Einlass 16 beaufschlagt wird. In der Fig. 10 nicht näher gezeigt ist der Auslass 18, der tangential im mittleren Bereich des Pumpengehäuses 10 entspringt (Fig. 3); in diesem Bereich der Pumpe sind sowohl der Druck als auch die Blutgeschwindigkeit am größten. Entsprechend sollte hier ein Stutzen für eine (nicht gezeigte) Ausflusskanüle von größtmöglichem Durchmesser sein, um eine Verlangsamung des Blutes zu gewährleisten; dies wird bevorzugt durch eine konische Form mit zunehmenden Querschnittsradien erreicht.

Das lediglich schematisch gezeigte Pumpengehäuse 10 in der Fig. 1 nimmt zusätzlich die Steuerelektronik für den Motor auf, wobei das System dann von einer implantierbaren Batterie mit Energie versorgt werden kann (besonders bevorzugt wird die Batterie mittels transkutanem Energietransfer geladen). Über diesen Versorgungsport können zudem aktuelle Betriebsdaten der Pumpe ausgelesen und zur externen Weiterverarbeitung geleitet werden, um die Pumpensteuerung zu beeinflussen. Weiterbildungsgemäß ist nämlich vorgesehen, die Förderleistung der Pumpe mittels elektromagnetischem Flussmesser zu kontrollieren, der bevorzugt von außen auf die Ausflusskanüle aufgebracht wird; ein weiterer Flussmesser misst den Blutfluss innerhalb der Pulmonalarterie. Die der Pumpe zugeordnete Steuereinheit stellt Flussdifferenzen fest und nutzt diese zur Regelung der Pumpe, so dass insbesondere Phänomene eines Overpumping mit Ansaugung der freien Ventrikelwand oder des Septums, oder Pumpenobstruktion, vermieden werden können.

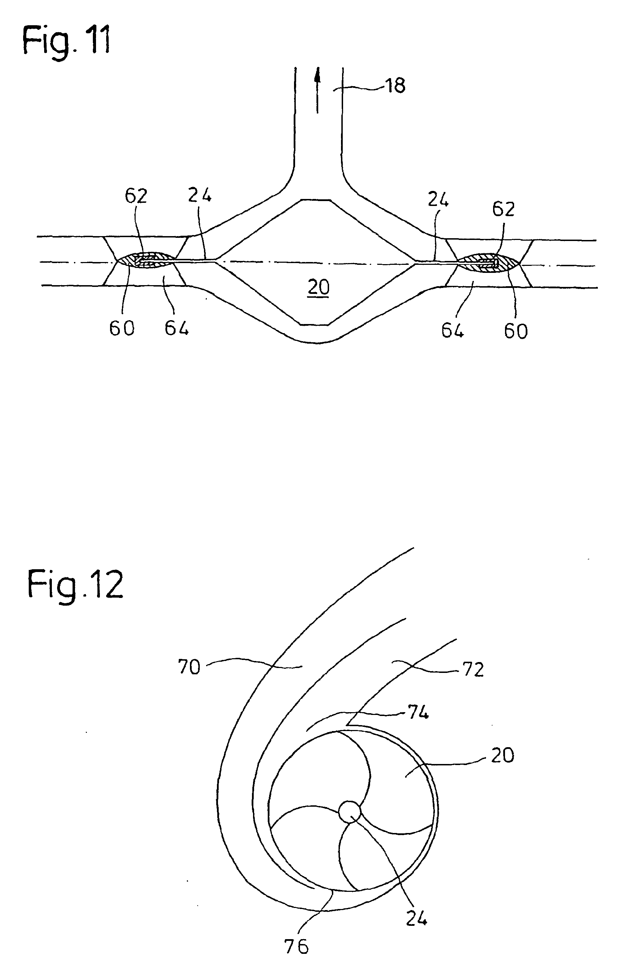

Die Fig. 11 zeigt eine alternative Ausbildung der endseitigen Lager für die Rotorachse bzw. Rotorwelle 24: Eingebettet jeweils in ein strömungsgünstig ausgebildetes, spitz zulaufendes Lagergehäuse 60 aus gut wärmeleitendem Material wird die Rotorwelle 24 in geeigneten Gleit- bzw. Wälzlagern 62 gelagert. In Richtung auf den Rotorköper ist jeweils eine (nicht gezeigte) Dichtung aus verschleißfreiem Material vorgesehen, wobei, bevorzugt, eine spitz auslaufende Dichtung direkt an der (zylindrischen) Welle anliegen kann. Durch geeignete Formgebung von Lagergehäuse und Dichtung kann dafür gesorgt werden, dass keine negative Beeinflussung der Blutströmung erfolgt, vielmehr wird diese bevorzugt dafür benutzt, in effektiver Weise das Lagergehäuse (und damit das Lager) zu kühlen.

Wie zudem in der Fig. 11 erkennbar ist, sind die jeweiligen Lagergehäuse 60 von einer Leiteinrichtung 64 gehalten, die aus drei innen, lang gestreckten und axial um die Rotationsachse herum angeordneten Flügeln besteht. Insbesondere dann, wenn diese Leiteinrichtung (in axialer und/oder in radialer Richtung) eine Krümmung aufweist, kann zudem das Einströmen des Blutes optimiert werden, da insoweit eine Vorrotation für den nachfolgenden Rotorkörper erfolgt. Durch den guten thermischen Kontakt mit dem Lagergehäuse dienen sie weiterhin als zusätzliche Kühlflächen für das Lager und verringern damit zusätzlich die örtlichen thermischen Belastungen des Blutes.

Fig. 12 zeigt, gegenüber Fig. 3, eine alternative Realisierung des Auslasses 18: Wie hier zu erkennen ist, sieht dieses Ausführungsbeispiel eine Mehrzahl von Auslasskanälen 70, 72 vor, die mit ihren jeweiligen Öffnungen 74, 76 zur Blutkammer hin um deren Umfang herum verteilt angeordnet sind. Dies führt dazu, dass der Rotor strömungstechnisch nicht lediglich an einer Umfangsstelle durch einen mit dem Auslass zusammenhängenden Strömungswiderstand belastet wird, sondern, wie in der Fig. 12 gezeigt, zwei oder gar mehr Öffnungen, bevorzugt um den Umfang herum verteilt, vorgesehen sind. Die einzelnen Auslasskanäle 70, 72 fließen stromabwärts dann geeignet zusammen.

Die vorliegende Erfindung ist nicht auf die beschriebenen Ausführungsbeispiele beschränkt, vielmehr sind weitere, bevorzugte Alternativen und/oder Weiterbildungen vorgesehen, die, praktisch bedeutsam, die atraumatischen Wirkungen der Pumpe weiter fördern. Hierzu gehört beispielsweise die querschnittliche Ausbildung der Rotorblätter 26, für die sich endseitig eine Rundung, insbesondere im vollen Radius, anbietet.

Eine weitere Variante der mechanisch wirksamen Lagerung der Rotorachse (Rotorwelle) besteht darin, dass die erfindungsgemäße Strömungsleiteinrichtung im Einflussstutzen eine feststehende Welle (genauer: Wellenansatz) beidseits der Blutkammer ausbildet, die jeweils in die Blutkammer hineinragt. Eine Spitze dieses feststehenden Wellenansatzes bildet dann ein Lagerteil für eine auf dem Rotor vorgesehene Lagerschale, die mit dem Wellenansatz zusammenwirkt.

Auf diese Weise wird eine mechanisch wirksame Lagerung realisiert, die in die Blutkammer hineinragen kann. Erreichbar ist damit, dass das Lager mit hohem Druck mit dem aus dem Einlass zufließenden Blut gespült werden kann; eine Spüllippe od.dgl. Leitelement ist nicht mehr notwendig.

Im Ergebnis wird mit der beschriebenen Geometrie damit nicht nur eine atraumatische, schonende Behandlung einfließenden Blutes (und damit eine weitgehende Verminderung von Thrombenbildung) erreicht, auch ist die haemodynamische Effizienz so hoch, dass Betriebsgeschwindigkeiten mit ca. 2000 U/min deutlich unterhalb der Rotationsgeschwindigkeiten existierender Pumpensysteme gehalten werden kann. Auch hierin drückt sich Langlebigkeit und Störungsunempfindlichkeit aus.

Während im beschriebenen Ausführungsbeispiel die Verschleiss unterliegende Lageranordnung aus Rotorwelle und Lagerschale üblicherweise aus hartem, abriebfestem Material gefertigt wird -- es bietet sich Edelstein für die Lagerschale und/oder die Spitze der Rotorwelle an, während die Welle selber typischerweise aus Keramik gefertigt ist -- liegt natürlich auch der Einsatz anderer geeigneter Materialien im Belieben des einschlägigen Fachmannes.

## Patentansprüche

1. Blutpumpe, insbesondere ventrikuläre Herzunterstützungspumpe, mit einer in einem Pumpengehäuse (10) gebildeten Blutkammer (12), in der ein um eine Rotorachse (24) drehbarer Rotor (20) aufgenommen ist, und die an einander gegenüberliegenden Enden jeweils einen mit einer Einflusskanüle (48, 50) verbindbaren Einlass (14, 16) ausbildet,
wobei ein mit einer Ausflusskanüle verbindbarer Auslass (18) der Blutkammer sich in einer Richtung senkrecht zur Rotorachse erstreckt,
**dadurch gekennzeichnet, dass**
die Rotorachse jeweils endseitig in mechanisch wirksamen Lagern (34, 40) drehbar gelagert ist, die im Bereich eines jeweiligen Einlasses (14, 16) vorgesehen und mit dem Pumpengehäuse verbunden sind.

2. Blutpumpe nach Anspruch 1, **dadurch gekennzeichnet, dass** die Blutkammer (12) als Doppelkonus ausgebildet ist, wobei im Bereich der auswärts weisenden Konusspitzen ein jeweiliger der Einlässe (14, 16) vorgesehen und der Auslass (18) im mittleren Bereich des Doppelkonus gebildet ist.

3. Blutpumpe nach Anspruch 1 oder 2, **dadurch gekennzeichnet, dass** eine lichte Innenweite der Blutkammer (12) zwischen einer Innenwand des Pumpengehäuses (10) und einer Außenfläche des Rotors (20) so gebildet und bemessen ist, dass in Richtung von einem jeweiligen Einlass (14, 16) auf den Auslass (18) eine für den Blutfluss wirksame Querschnitts- bzw. Durchtrittsfläche zwischen Pumpengehäuse und Rotor sich bevorzugt kontinuierlich verringert.

4. Blutpumpe nach einem der Ansprüche 1 bis 3, **dadurch gekennzeichnet, dass** die mechanisch wirksamen Lager zusätzlich magnetisch wirksame Lagermittel aufweisen, die eine zusätzliche Führung oder Lagerung und/oder Unterstützung der mechanisch wirksamen Lager bewirken.

5. Blutpumpe nach einem der Ansprüche 1 bis 4, **gekennzeichnet durch** Mittel (36) zur Flussführung, eingeschlossen Wirbelverhinderung, Flussbegradigung oder Vorrotation, von in einen Einlass einfließenden Blutes, die mit dem Pumpengehäuse verbunden sind und zusätzlich die Lager (40) für die Rotorachse ausbilden.

6. Blutpumpe nach einem der Ansprüche 1 bis 5, **dadurch gekennzeichnet, dass** die Lager (40) für die Rotorachse (24) so ausgebildet sind, dass sie durch in die Einlässe (14, 16) einfließendes Blut spülbar sind, wobei bevorzugt die Lager eine in den Fluss des einfließenden Blutes hineinreichende Spüllippe (44) oder dergleichen Leitelement aufweisen.

7. Blutpumpe nach einem der Ansprüche 1 bis 6, **gekennzeichnet durch** Mittel zur Druck-, Volumenstrom- und/oder Flussmessung von einfließendem sowie ausfließendem Blut, die zur Erzeugung eines Steuersignals für eine Steuerung des Pumpenantriebes, insbesondere Drehzahlsteuerung nach einem vorbestimmten Zusammenhang mit einer Pumpenleistung, ausgebildet sind, wobei bevorzugt zusätzlich Mittel zur Erfassung einer Rotordrehzahl und/oder einer elektrischen Antriebsleistung der Blutpumpe vorgesehen sind.

8. Blutpumpe nach einem der Ansprüche 1 bis 7, **dadurch gekennzeichnet, dass** eine der Blutpumpe zugeordnete elektronische Steuereinheit zum Erfassen von EKG-Signalen, und insbesondere zum Zusammenwirken mit einer Herzschrittmachereinheit, ausgebildet ist.

9. Blutpumpe nach einem der Ansprüche 1 bis 8, **dadurch gekennzeichnet, dass** der Auslass der Blutkammer eine Mehrzahl von in Umfangsrichtung des Rotors zumindest abschnittsweise parallel verlaufenden Auslasskanälen (70, 72) aufweist, die jeweils eine umfangsseitig verteilt angeordnete Öffnung (74, 76) zur Blutkammer aufweisen.

10. Blutpumpe nach einem der Ansprüche 1 bis 9, **dadurch gekennzeichnet, dass** die mechanisch wirksamen Lager (62) ein bevorzugt gegen einfließendes Blut gedichtetes Lagergehäuse (60) aufweisen, welches so ausgebildet ist, dass die Lager durch das einfließende Blut gekühlt werden und/oder eine atraumatische Umströmung mit dem einfließenden Blut erreicht wird.

11. Blutpumpe nach einem der Ansprüche 1 bis 10, **dadurch gekennzeichnet, dass** zum Antrieb des Rotors eine elektrisch ansteuerbare Spulenvorrichtung vorgesehen ist, die eine Mehrzahl von separat, insbesondere zu Zwecken der Redundanz oder Leistungssteuerung ansteuerbaren Wicklungen aufweist.

## Claims

1. Blood pump, in particular ventricular cardiac support pump, with formed in a pump housing (10) a blood chamber (12) which is designed to hold a rotor (20) rotatable about a rotor axis (24) and at opposite ends in sections forms an inlet (14, 16) which can be connected with an inlet cannula (48, 50) in each case, where an outlet (18) from the blood chamber which can be connected with an outlet cannula extends in a direction perpendicular to the rotor axis, **characterised in that** the rotor axis at its ends is rotatably mounted in mechanically active bearings (34, 40) which are provided in the area of each inlet (14, 16), and are connected with the pump housing.

2. Blood pump according to claim 1, **characterised in that** the blood chamber (12) is formed as a double cone, where in the area of each outward pointing cone tip is provided an inlet (14, 16), and the outlet (18) is formed in the middle area of the double cone.

3. Blood pump according to claim 1 or 2, **characterised in that** the clear internal width of the blood chamber (12) between an inner wall of the pump housing (10) and an outer surface of the rotor (20) is formed and dimensioned such that in the direction from each inlet (14, 16) to the outlet (18), a cross-section or passage surface active for the blood flow between the pump housing and rotor diminishes preferably continuously.

4. Blood pump according to any of claims 1 to 3, **characterised in that** the mechanically active bearings also have magnetically active bearing means which achieve an additional guidance or mounting and/or support for the mechanically active bearings.

5. Blood pump according to any of claims 1 to 4, **characterised by** means (36) for flow guidance including turbulence prevention, flow regulation or prerotation of blood flowing into an inlet, which means are connected with the pump housing and also form the bearing (40) for the rotor axis.

6. Blood pump according to any of claims 1 to 5, **characterised in that** the bearings (40) for the rotor axis (24) are formed so that they can be flushed by blood flowing in through the inlets (14, 16), where preferably the bearing has a flushing lip (44) or similar guide element projecting into the flow of the incoming blood.

7. Blood pump according to any of claims 1 to 6, **characterised by** means for measuring the pressure, volume flow and/or flow of incoming or outgoing blood which are designed to generate a control signal for control of a pump drive, in particular rotation speed control, according to a preset correlation with a pump output, where preferably in addition means are provided for detecting a rotor rotation speed and/or an electrical drive power of the blood pump.

8. Blood pump according to any of claims 1 to 7, **characterised in that** an electronic control unit allocated to the blood pump is designed to detect to ECG signals, in particular for co-operation with a cardiac pacemaker unit.

9. Blood pump according to any of claims 1 to 8, **characterised in that** the outlet from the blood chamber has a multiplicity of outlet channels (70, 72) running parallel at least in sections in the peripheral direction of the rotor, each of which has an opening (74, 76) to the blood chamber arranged distributed about the periphery.

10. Blood pump according to any of claims 1 to 9, **characterised in that** the mechanically active bearings (62) have a bearing housing (60) preferably sealed against the incoming blood which is formed so that the bearings are cooled by the incoming blood and/or an atraumatic flow is achieved with the incoming blood.

11. Blood pump according to any of claims 1 to 10, **characterised in that** to drive the rotor, an electrically controllable coil device is provided which has a multiplicity of windings separately controllable in particular for the purposes of redundancy or power control.

## Revendications

1. Pompe à sang, en particulier pompe d'assistance cardiaque ventriculaire, comprenant une chambre à sang (12) formée dans un boîtier de pompe (10), chambre dans laquelle un rotor (20), adapté à tourner autour d'un axe de rotor (24), est accueilli et chambre qui forme une entrée (14, 16), adaptée à être reliée avec une canule d'entrée (48, 50), respectivement à des extrémités opposées l'une à l'autre, une sortie (18) de la chambre à sang, adaptée à être reliée avec une canule de sortie, s'étendant dans une direction perpendiculaire à l'axe de rotor,
**caractérisée en ce que**
- l'axe de rotor est monté de façon rotative respectivement du côté des extrémités dans des paliers (34, 40) efficaces mécaniquement, les paliers étant prévus dans la région d'une entrée (14, 16) respective et étant reliés au boîtier de pompe.

2. Pompe à sang selon la revendication 1, **caractérisée en ce que** la chambre à sang (12) à la forme d'un double-cône, l'une des entrées (14, 16) étant respectivement prévue dans la région de la pointe du cône orientée vers l'extérieur et la sortie (18) étant formée dans une zone médiane du double-cône.

3. Pompe à sang selon la revendication 1 ou 2, **caractérisée en ce qu'**un diamètre intérieur clair de la chambre à sang (12) entre une paroi intérieure du boîtier de pompe (10) et une surface extérieure du rotor (20) est formé et déterminé de telle sorte que, en direction partant d'une entrée (14, 16) respective vers la sortie (18), une surface transversale ou de passage efficace pour le flux de sang se rétrécit de préférence de façon continue entre le boîtier de pompe et le rotor.

4. Pompe à sang selon l'une des revendications 1 à 3, **caractérisée en ce que** les paliers efficaces mécaniquement comprenant des moyens de palier supplémentaires efficaces magnétiquement, qui produisent un guidage, placement et/ou un soutien des paliers efficaces mécaniquement.

5. Pompe à sang selon l'une des revendications 1 à 4, **caractérisée par** des moyens (36) pour guider le flux, pour empêcher un tourbillon d'être enfermé, pour rectifier le flux ou pour une prérotation, à partir de sang entré par une entrée, moyens qui sont reliés au boîtier de pompe et forment en outre les paliers (40) pour l'axe de rotor.

6. Pompe à sang selon l'une des revendications 1 à 5, **caractérisée en ce que** les paliers (40) pour l'axe de rotor (24) sont formés de telle sorte qu'ils sont adaptés à être balayés par du sang entré par les entrées (14, 16), les paliers présentant de préférence une lèvre de balayage (44) passant dans le flux du sang entrant, ou tout autre élément de guidage équivalent.

7. Pompe à sang selon l'une des revendications 1 à 6, **caractérisée par** des moyens de mesure de la pression, de mesure du flux volumique et/ou de mesure du flux à partir du sang entrant et sortant, moyens qui sont formés pour générer un signal de commande du dispositif d'entraînement de la pompe, en particulier une commande de vitesse de rotation selon un rapport prédéterminé avec un rendement de pompe, les moyens étant en outre de préférence prévus pour détecter une vitesse de rotation de rotor et/ou un rendement d'entraînement électrique de la pompe à sang.

8. Pompe à sang selon l'une des revendications 1 à 7, **caractérisée en ce qu'**une unité de commande électronique associé à la pompe à sang est formée pour détecter des signaux d'ECG et en particulier pour coopérer avec une unité de stimulateur cardiaque.

9. Pompe à sang selon l'une des revendications 1 à 8, **caractérisée en ce que** la sortie de la chambre à sang comprend une pluralité de canaux de sortie (70, 72) courant parallèlement en direction circonférentielle du rotor au moins en coupe, canaux de sortie qui comprennent chacun une ouverture (74, 76) répartie du côté de la circonférence et menant vers la chambre à sang.

10. Pompe à sang selon l'une des revendications 1 à 9, **caractérisée en ce que** les paliers (62) efficaces mécaniquement comprennent un boîtier de palier (60) de préférence étanche au sang entrant, boîtier de palier qui est formé de telle sorte que les paliers sont refroidis par le sang entrant et/ou de telle sorte qu'un écoulement atraumatique soit atteint avec le sang entrant.

11. Pompe à sang selon l'une des revendications 1 à 10, **caractérisée en ce que**, pour l'entraînement du rotor, un dispositif à bobinage électrique adapté à subir une commande d'amorçage, dispositif qui comprend une pluralité de bobinages séparés adaptés à subir une commande d'amorçage, en particulier dans des buts de redondance ou de commande du rendement.
